**Europäisches Patentamt**

**European Patent Office**

**Office européen des brevets**

(11) Veröffentlichungsnummer: **0 460 257 A1**

# EUROPÄISCHE PATENTANMELDUNG

(21) Anmeldenummer: **90110710.2**

(51) Int. Cl.5: **H01H 3/14**

(22) Anmeldetag: **06.06.90**

(43) Veröffentlichungstag der Anmeldung:
**11.12.91 Patentblatt 91/50**

(84) Benannte Vertragsstaaten:
**DE FR GB**

(71) Anmelder: **SIEMENS AKTIENGESELLSCHAFT**
**Wittelsbacherplatz 2**
**W-8000 München 2(DE)**

(72) Erfinder: **Amtmann, Heribert, Dipl.-Ing. (FH)**
**Zedernstrasse 15**
**W-8521 Langensendelbach(DE)**

(54) **Fusssteuerungsvorrichtung.**

(57) Es soll eine Fußsteuerungsvorrichtung geschaffen werden, durch die eine zweidimensionale Steuerung eines Anwahl- und/oder Verstellvorganges und zusätzlich weitere Quittier- und Steuerbefehle möglich sind. Die Fußsteuerungsvorrichtung soll insbesondere zur Steuerung einer Röntgenanlage anwendbar sein.

Es sind mehrere drehbare Walzen (2 bis 5) vorhanden, welche Signalgeber zur Erzeugung von der jeweiligen Walzenstellung entsprechenden elektrischen Signalen betätigen und welche durch Fußdruck abbremsbar und stillsetzbar sind.

FIG 1

EP 0 460 257 A1

Fußsteuerungsvorrichtungen werden in der Technik für die vielfältigsten Zwecke verwendet. So ist es z.B. durch die DE-OS 20 10 360 bekannt, bei einer Röntgenanlage einen Fußschalter zur Dosisleistungssteuerung zu verwenden. Die bekannte Fußsteuerungsvorrichtung erlaubt demgemäß nur die Auslösung von Schaltvorgängen.

Der Erfindung liegt die Aufgabe zugrunde, eine Fußsteuerungsvorrichtung zu schaffen, die die kontinuierliche Steuerung von Bewegungen erlaubt und deshalb insbesondere in der Röntgentechnik einsetzbar ist.

Diese Aufgabe ist erfindungsgemäß gelöst durch mindestens eine drehbare Walze, welche einen Signalgeber zur Erzeugung eines der jeweiligen Walzenstellung entsprechenden elektrischen Signales betätigt und welche durch Fußdruck gebremst wird und stillsetzbar ist. Wird durch den Signalgeber eine verstellbare Komponente angesteuert und werden mehrere Walzen mit ihren Längsachsen entsprechend angeordnet, so ist es möglich, durch geeignete Bremsung der Walzen eine zweidimensionale Bewegungssteuerung vorzunehmen.

Die Erfindung ist nachfolgend anhand eines in der Zeichnung dargestellten Ausführungsbeispieles näher erläutert. Es zeigen:

Fig. 1 eine Fußsteuerungsvorrichtung nach der Erfindung,

Fig. 2 und 3 Einzelheiten der Fußsteuerungsvorrichtung nach Fig. 1,

Fig. 4 eine durch die Fußsteuerungsvorrichtung gemäß den Fig. 1 bis 3 steuerbare Röntgenanlage, und

Fig. 5 ein Blockschaltbild für die Fußsteuerungsvorrichtung nach den Fig. 1 bis 4.

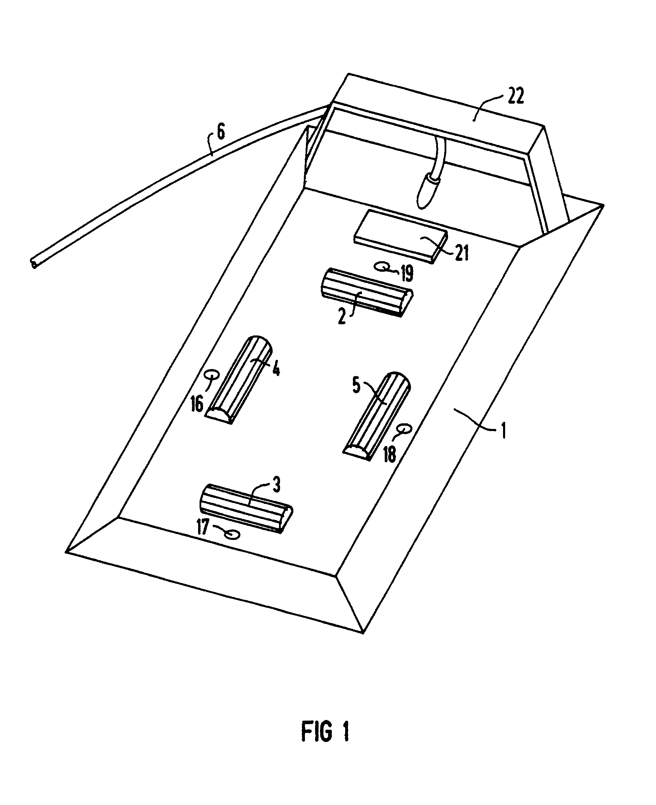

In der Fig. 1 ist mit 1 das Gehäuse einer Fußsteuerungsvorrichtung bezeichnet, in dem vier Walzen 2, 3, 4, 5 drehbar gelagert sind, welche aus der Oberfläche des Gehäuses 1 etwas herausragen. Die Oberfläche der Walzen 2 bis 5 ist geriffelt oder mit Noppen versehen, so daß ein guter Kontakt zum Schuh der Bedienungsperson möglich ist. Ein Kabel 6 führt zu einer zugeordneten Elektronik.

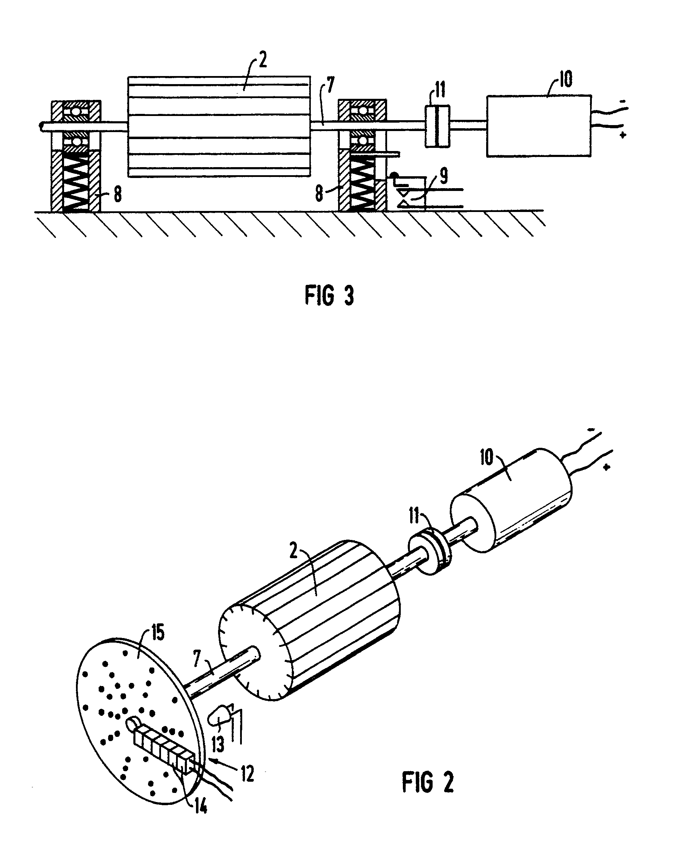

Die Fig. 2 und 3 zeigen die Walze 2, welche mit ihrer Achse 7 in zwei Haltern 8 senkrecht zu der Achse 7 federnd gelagert ist. Wird ein Druck auf die Walze 2 von oben ausgeübt, so bewegt sich diese nach unten und betätigt einen Schalter 9. Die Walze 2 wird von einem Elektromotor 10 über eine Rutschkupplung 11 angetrieben und dreht ihrerseits gemäß Fig. 2 einen elektro-optischen Signalgeber 12 mit einer Leuchtdiode 13, Fototransistoren 14 und einer Codierscheibe 15. Die Fototransistoren 14 erhalten von der Leuchtdiode 13 Licht entsprechend der auf der Codierscheibe 15 vorgesehenen Codierung, so daß ihre Ausgangssignale von der jeweiligen Stellung der Codierscheibe 15 und damit der Walze 2 abhängen.

Die Walzen 3, 4, 5 sind analog zur Walze 2 gelagert und angetrieben sowie mit entsprechenden Signalgebern versehen.

Drückt die Bedienungsperson mit ihrem Fuß auf die Walzen 2 bis 5, so bremst sie die Walze bzw. setzt diese still und betätigt den jeweiligen Schalter 9. Dadurch wird die jeweilige Position der Walze 2 bis 5 an ein zu steuerndes Gerät weitergegeben und durch den Schalter 9 quittiert.

Durch die beschriebene Fußsteuerungsvorrichtung werden folgende Anforderungen erfüllt:

1. Die Bedienungsperson kann im Stehen arbeiten.

2. Sie ist insgesamt leicht mit dem Fuß verschiebbar.

3. Die Steuerungselemente (Walzen 2 bis 5) reagieren auf Druck (Gaspedalfunktion).

4. Steuerungsbefehle sind durch unterschiedliches Schuhwerk gebbar.

5. Steuerungsbefehle sind ohne Sichtkontakt mit der Fußsteuerungsvorrichtung gebbar.

6. Die Hände der Bedienungsperson sind frei.

7. Das Anlernen ist schnell und einfach möglich.

8. Die Funktionen einer "Mausbedienung" können voll nachgebildet werden.

Die Fig. 1 zeigt, daß neben den Walzen 2 bis 5 Leuchtdioden 16 bis 19 angeordnet sind. Dadurch ist ein Auffinden der Walzen 2 bis 5 auch im Dunklen leicht möglich. Die Leuchtdioden 16 bis 19 können hierzu verschiedene Farben besitzen.

Das Kabel 6 kann durch eine drahtlose Fernsteuerung, z.B. durch eine Infrarotübertragungsstrecke, ersetzt werden.

Anstelle der motorischen Drehung der Walzen 2 bis 5 ist es auch möglich, auf einen motorischen Antrieb zu verzichten. Wird die Schwungmasse der Walzen 2 bis 5 ausreichend bemessen, so ist eine Drehung der Walzen 2 bis 5 über den Fuß möglich.

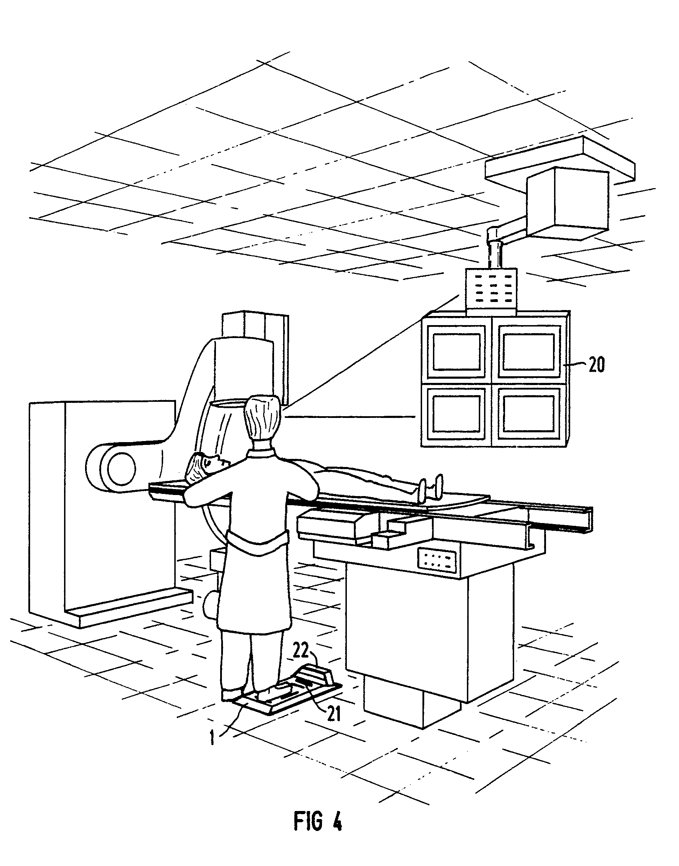

Die Fig. 4 zeigt die Anwendung der Fußsteuerungsvorrichtung gemäß den Fig. 1 bis 3 in Verbindung mit einer Röntgenanlage. Die Bedienungsperson hat die Hände für Manipulationen am Patienten frei und kann Steuerungsbefehle, z.B. zur Bewegung der Tischplatte, geben, ohne den Blick auf die Fußsteuerungsvorrichtung zu richten. Sie kann demgemäß dabei z.B. ein Anzeigetableau 20 im Auge behalten.

Die Fig. 1 zeigt, daß die Fußsteuerungsvorrichtung einen Fußschalter 21 aufweist, der durch Druck betätigbar ist und durch den z.B. eine Röntgenaufnahme ausgelöst werden kann. Weitere entsprechende Fußschalter können am Gehäuse 1 angeordnet sein und z.B. die Funktionen "Durchleuchtung ein/aus, Durchleuchtungsebene A/B" haben.

Ein Bügel 22 am Gehäuse 1 erlaubt die leichte Verstellung der Fußsteuerungsvorrichtung mit dem Fuß.

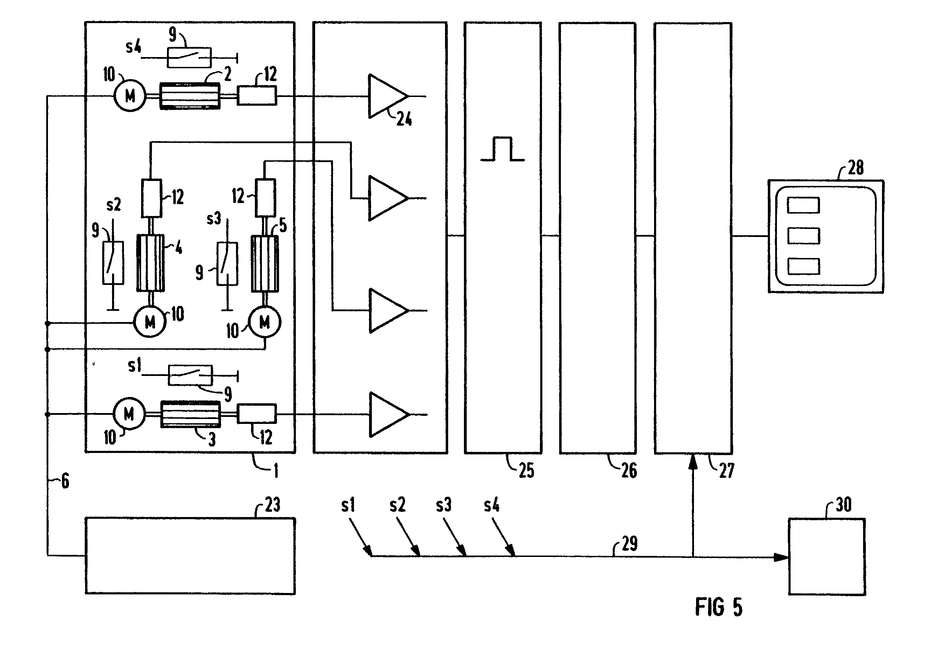

Die Fußsteuerungsvorrichtung eignet sich insbesondere bei einer Röntgenanlage gemäß Fig. 4 für die interventionelle Technik, da dabei die Hände des Arztes für die Hantierung des Katheters belegt sind. Der Arzt steht während der Untersuchung und die Walzen 2 bis 5 ermöglichen es, daß sich der Arzt immer mit dem Absatz oder mit dem Ballen abstützt. Durch den Schuh wird Druck auf die Walzen 2 bis 5 ausgeübt und es ist ein genau dosiertes Abbremsen möglich. Es ist auch möglich, zwei Walzen 2 bis 5 gleichzeitig zu bremsen, so daß z.B. eine Schrägbewegung des Cursors auf dem zugeordneten Anzeigetableau 28 in Fig. 5 möglich ist.

Die Fig. 5 zeigt das Gehäuse 1, die Walzen 2 bis 5, die Schalter 9, die Motoren 10 und die Signalgeber 12 schematisch. Über das Kabel 6 ist eine Spannungsversorgung 23 angeschlossen.

Die Ausgangssignale der Signalgeber 12 werden über Meßverstärker 24, einen Impulsformer 25 und einen Prozessor 26 einem Computer 27 zugeführt, der ein Anzeigetableau 28 steuert. Die Signale der Schalter 9 bewirken über eine Leitung 29 eine Fernsteuerung der schematisch dargestellten Röntgenanlage 30 gemäß Fig. 4. Über die Schalter 9 kann z.B. auch auf Durchleuchtung oder Aufnahme geschaltet werden. Die Signale der Signalgeber 12 bewirken über den Prozessor 27 ebenfalls eine Steuerung der Röntgenanlage 30, z.B. der Tischplatte.

Die beschriebene Fußsteuerungsvorrichtung ist insbesondere in der Röntgentechnik, aber auch für andere Steuerungsaufgaben, z.B. für graphische Work-Stationen, als Bedienelement für Arm- und Hand-Behinderte, in der allgemeinen Steuerungs- und Überwachungstechnik und in der Verkehrstechnik anwendbar.

**Patentansprüche**

1. Fußsteuerungsvorrichtung mit mindestens einer drehbaren Walze (2 bis 5), welche einen Signalgeber (12) zur Erzeugung eines der jeweiligen Walzenstellung entsprechenden elektrischen Signales betätigt und welche durch Fußdruck gebremst wird und stillsetzbar ist.

2. Fußsteuerungsvorrichtung nach Anspruch 1, bei der die Walze (2 bis 5) senkrecht zu ihrer Längsachse federnd gelagert ist und bei Verstellung in dieser Richtung Schaltmittel (9) zur Signalgabe betätigt.

3. Fußsteuerungsvorrichtung nach Anspruch 1 oder 2, bei der vier Walzen (2 bis 5) so angeordnet sind, daß ihre Achsen ein Rechteck bilden, so daß eine zweidimensionale Verstellbewegung steuerbar ist.

4. Fußsteuerungsvorrichtung nach einem der Ansprüche 1 bis 3, **gekennzeichnet durch** die Anwendung für die Steuerung einer Röntgenanlage (30).

FIG 1

FIG 3

FIG 2

FIG 4

EP 0 460 257 A1

FIG 5

**Europäisches Patentamt**

**EUROPÄISCHER RECHERCHENBERICHT**

Nummer der Anmeldung

**EP 90 11 0710**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | FR-A-2 392 654 (EMDA) <br> * Seite 5, Zeile 6 - Seite 6, Zeile 18; Figuren 1-3 * <br> – – – | 1 | H 01 H 3/14 |
| Y | FR-A-2 425 841 (TOKYO ENGINE KOGYO K.K.) <br> * Ansprüche 1, 2 * <br> – – – | 1 | |
| A | FR-A-1 157 008 (ETABLISSEMENTS ALEXANDRE) <br> * Seite 2, Spalte 2, Absatz 7 - Seite 3, Spalte 1, Absatz 3; Figur 1 * <br> – – – | 1 | |
| D,A | FR-A-2 084 166 (SIEMENS) <br> * Figur * <br> – – – – – | 1 | |

|  |  |  | RECHERCHIERTE SACHGEBIETE (Int. Cl.5) |
|---|---|---|---|
|  |  |  | H 01 H <br> G 05 G <br> A 61 C |

Der vorliegende Recherchenbericht wurde für alle Patentansprüche erstellt

| Recherchenort | Abschlußdatum der Recherche | Prüfer |
|---|---|---|
| Den Haag | 08 Februar 91 | JANSSENS DE VROOM P. |